(19) Europäisches Patentamt
European Patent Office
Office européen des brevets

(11) Publication number: **0 452 507 A1**

(12) **EUROPEAN PATENT APPLICATION published in accordance with Art. 158(3) EPC**

(21) Application number: **90916355.2**

(51) Int. Cl.5: **A61K 31/70**, //C07H17/065

(22) Date of filing: **08.11.90**

(86) International application number: **PCT/JP90/01450**

(87) International publication number: **WO 91/07179 (30.05.91 91/12)**

(30) Priority: **08.11.89 JP 290806/89**
**08.11.89 JP 290807/89**
**08.11.89 JP 290808/89**

(43) Date of publication of application: **23.10.91 Bulletin 91/43**

(84) Designated Contracting States: **CH DE ES FR GB IT LI**

(71) Applicant: **NIPPON HYPOX LABORATORIES INCORPORATED**
**1759, Matsugaya Hachioji-shi**
**Tokyo 192-03(JP)**

(72) Inventor: **SATOH, Toshio**
**57-3, Nagao Jorokucho**
**Tokushima-shi Tokushima-ken 771-42(JP)**
Inventor: **MATSUMOTO, Hitoshi**
**125-22, Shimofukuman Hachiman-cho**
**Tokushima-shi Tokushima-ken 770(JP)**
Inventor: **KEKEGAWA, Hisao, 404 Dia Palace Nakamaekawa**
**5-1-222, Nakamaekawa-cho**
**Tokushima-shi, Tokushima-ken 770(JP)**
Inventor: **NIIRO, Yasunori**
**4-33-206, Minamisuehiro-cho Tokushima-shi**
**Tokushima-ken 770(JP)**

(74) Representative: **Türk, Dietmar, Dr. rer. nat. et al**
**Türk, Gille + Hrabal Patentanwälte**
**Brucknerstrasse 20**
**W-4000 Düsseldorf 13(DE)**

(54) **MEDICINE COMPRISING TOCOPHERYL GLYCOSIDE AS ACTIVE INGREDIENT.**

(57) A preventive and therapeutic agent for delayed hypersensitivity, an immunoglobulin E antibody production inhibitor and an antiinflammatory drug comprising α-tocopheryl glycoside as an active ingredient.

EP 0 452 507 A1

## TECHNICAL FIELD

The present invention relates to a medicament containing tocopheryl glycoside as an active component, particularly to a preventive and therapeutical preparation for delayed hypersensitivity, an immunoglobulin E antibody production inhibiting preparation and an anti-inflammatory preparation.

## TECHNICAL BACKGROUND

(1) Delayed-type hypersensitivity diseases such as contact dermatitis, atopic dermatitis, nephritis, etc., are caused by a delayed-type hypersensitivity reaction based on cellular immunity, and therefore cannot be prevented or cured by medicaments used for the therapy of immediate-type hypersensitivity diseases such as an antihistamic preparation, an antiallergic preparation, etc.

For the therapy of the above delayed-type hypersensitivity diseases, therefore, a steroid preparation containing cortisone, hydrocortisone, predonisolone, or the like as a main component, or an immunosuppressive preparation containing methotrexate, azathiouprine, or the like as a main component is conventionally used.

Since, however, the therapy of the delayed-type hypersensitivity diseases with the conventional steroid or immunosuppressive preparation is a nosotropic one, and produces a high side effect, such a preparation cannot be administered in a large amount or continuously. Therefore, there has been a problem in that it is difficult to obtain a high therapeutical effect without producing a side effect.

It is therefore a first object of the present invention to provide a preventive and therapeutical preparation for delayed-type hypersensitivity, of which the side effect is low and which has an excellent preventive and therapeutical effect on delayed-type hypersensitivity.

(2) Immunoglobulin E antibody (to be referred to as "IgE antibody" hereinafter) is an antibody which relates to anaphylaxis, and it is considered that diseases such as pollinosis, rhinitis, allergic brochitis, atopic dermatitis, etc., are caused since an excess amount of histamine is released from mast cells and basophils due to a coupling between this IgE antibody and an antigen (to be referred to as "IgE antibody-antigen mediating immune response).

In the therapy of disease caused by such an IgE antibody-antigen mediating immune response, it is conventional practice to use an antiallergic preparation which inhibits the release of chemical mediators such as histamine, leucotriene (SRS-A), etc., from mast cells and basophils, an antihistamine preparation ($H_1$ antagonist) which inhibits the coupling of free histamine with an $H_1$ receptor, an adrenocortical hormone preparation such as hydrocortisone, etc., or the like.

However, medicaments such as antiallergic preparations, antihistamine preparations, adrenocortical hormone preparations, etc., are medicaments for nosotropic therapy, and do not mainly have the activity to inhibit the production of IgE antibody which relates to anaphylaxis. There have been therefore a problem in that the above conventional medicaments cannot radically cure diseases caused by the IgE antibody-antigen mediating immune response.

Therefore, it is a second object of the present invention to provide an IgE antibody production inhibiting preparation, which can radically cure diseases caused by the IgE antibody-antigen mediating immune response.

(3) As an anti-inflammatory preparation, nonsteroidal anti-inflammatory preparations such as aspirin, indomethacin, etc., and steroidal anti-inflammatory preparations such as hydrocortisone, predonisolone, etc., have been developed, and have been and are widely used for the clinical therapy of various inflammations.

Since, however, the nonsteroidal anti-inflammatory preparations exhibit the anti-inflammatory activity by suppressing the biosynthesis of prostaglandins from arachidonic acid by means of the inhibition of cyclooxygenase, and since the steroidal anti-inflammatory preparations exhibit the anti-inflammatory activity by suppressing the release of arachidonic acid by means of the inhibition of phospholipase $A_2$, the nonsteroidal anti-inflammatory and steroidal anti-inflammatory preparations have a problem in that they produce a severe side effect of ulceration, although these preparations have strong anti-inflammatory activities.

Further, it is known that there are inflammations on which conventional nonsteroidal anti-inflammatory and steroidal anti-inflammatory preparations scarcely have a preventive and therapeutical effect.

It is therefore a third object of the present invention to provide an anti-inflammatory preparation which has no side effect on the digestive system and has wide anti-inflammatory activities.

## DISCLOSURE OF THE INVENTION

In order to achieve the above first object, the present inventors have studied physiological activities of a variety of substances, and found that substances classified as α-tocopheryl glycoside show a low side effect and have excellent preventive and therapeutical effects on delayed-type hypersensitivity diseases. The present invention has been completed on the basis of this finding.

That is, the preventive and therapeutical preparation for delayed-type hypersensitivity, provided by the present invention, contains, as an active component, at least one substance selected from α-tocopheryl glycosides.

Further, in order to achieve the above second object, the present inventors have studied physiological activities of a variety of substances, and found that substances classified as α-tocopheryl glycoside show a low side effect and have excellent IgE antibody production inhibition activities. The present invention has been completed on the basis of this finding.

That is, the IgE antibody production inhibiting preparation of the present invention contains, as an active component, at least one substance selected from α-tocopheryl glycosides.

Furthermore, in order to achieve the above third object, the present inventors have studied physiological activities of a variety of substances, and found that substances classified as α-tocopheryl glycoside have no side effect on the digestive system and exhibit excellent anti-inflammatory activities widely to inflammations including those which are difficult to prevent and cure with nonsteroidal anti-inflammatory preparations or sterodial anti-inflammatory preparations. The present invention has been completed on the basis of this finding.

That is, the anti-inflammatory preparation of the present invention contains, as an active component, at least one substance selected from α-tocopheryl glycosides.

BRIEF DESCRIPTION OF DRAWINGS

Fig. 1 is a graph showing pharmacological efficacies of a preventive and therapeutical preparation for delayed-type hypersensitivity in one Example of the present invention.

Fig. 2 is a graph showing pharmacological efficacies of a preventive and therapeutical preparation for delayed-type hypersensitivity in other Example of the present invention.

Fig. 3 is a graph showing one embodiment of pharmacological efficacy of an anti-inflammatory preparation of the present invention against an antigen-adjuvant-induced inflammation.

Fig. 4 is a graph showing other embodiment of pharmacological efficacy of an anti-inflammatory preparation of the present invention against an antigen-adjuvant-induced inflammation.

Fig. 5 is a graph showing one embodiment of pharmacological efficacy of an anti-inflammatory preparation of the present invention against adjuvant arthritis.

Fig. 6 is a graph showing one embodiment of pharmacological efficacy of an anti-inflammatory preparation of the present invention against carrageenin-induced inflammation.

Fig. 7 is a graph showing one side effect test of an anti-inflammatory preparation of the present invention.

PREFERRED EMBODIMENTS FOR WORKING THE INVENTION

Each of the preventive and therapeutical preparation for delayed-type hypersensitivity, the IgE antibody production inhibiting preparation and the anti-inflammatory preparation, provided by the present invention, contains, as an active component, at least one substance selected from α-tocopheryl glycosides.

The "at least one substance" selected from α-tocopheryl glycosides and contained as an active component in the preventive and therapeutical preparation for delayed-type hypersensitivity, IgE antibody production inhibiting preparation and anti-inflammatory preparation of the present invention is selected from d-types, l-types and dl-types of various substances such as α-tocopheryl mannoside, α-tocopheryl glucoside, α-tocopheryl galactoside, α-tocopheryl fucoside, α-tocopheryl xyloside, α-tocopheryl rhamnoside, etc. In the preventive and therapeutical preparation for delayed-type hypersensitivity and the IgE antibody production inhibiting preparation, provided by the present invention, it is particularly preferred to use dl-α-tocopheryl-α-D-mannoside (to be referred to as VIE-MAN hereinafter) and dl-α-tocopheryl-β-D-glucoside (to be referred to as VIE-GLU hereinafter).

VIE-MAN is a known low-toxicity substance which is represented by the formula

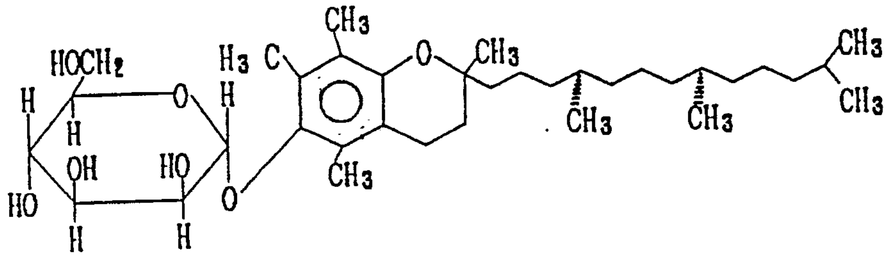

and obtained by reacting tocopherol (vitamin E) with D-mannose.

VIE-GLU is also a known low-toxicity substance which is represented by the formula

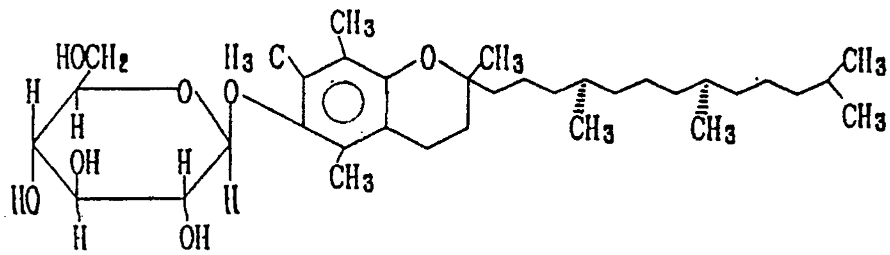

and obtained by reacting tocopherol (vitamin E) with D-glucose.

In the anti-inflammatory preparation of the present invention, it is particularly preferred to use the above VIE-MAN, the above VIE-GLU and dl-α-tocopheryl-β-galactoside (to be referred to as VIE-GAL hereinafter).

VIE-GAL is a known low-toxicity substance which is represented by the formula

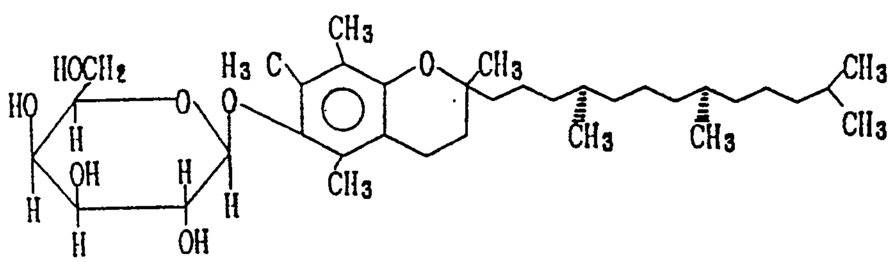

and obtained by reacting tocopherol (vitamin E) with D-galactose.

As described above, the preventive and therapeutical preparation for delayed-type hypersensitivity, the IgE antibody production inhibiting preparation and the anti-inflammatory preparation, provided by the present invention, contain, as an active component, at least one substance selected from α-tocopherol glycosides. The form of these preparations is selected from forms of orally administered solid preparations such as powders, grains, granules, tablets, coated tablets, capsules, etc., orally administered liquid preparations such as syrup, etc., transdermal absorption preparations, injections, suppositories, preparations for oral use, preparations for the eye, etc. The preparations of the present invention can be formed directly or in the presence of a conventional preparation carrier according to any one of conventional methods.

The dose of each of the preventive and therapeutical preparation for delayed-type hypersensitivity, the IgE antibody production inhibiting preparation and the anti-inflammatory preparation, provided by the present invention, differs depending upon kind and degree of diseases, preparation form, age and health state of a patient, etc., and therefore, cannot be fixed. In general, however, each of the above preparations of the present invention can be administered at a dose that the amount of at least one substance selected from α-tocopheryl glycosides as an active component is 1 to 5,000 mg/time/patient, whereby the desired effect can be obtained.

[Examples]

Examples of the present invention will be described hereinbelow.

Example 1 and Comparative Example 1

76 Male mice (C57BL/6Ncrj) weighing 18 to 20 g were used. The abdominal portion of each mouse was shaved such that the shaved portion was about 1 $cm^2$. Thereafter, 20 $\mu l$ of an ethanol solution of 7 % picryl chloride was applied to the shaved portions for sensitization.

Eighteen out of the 76 mice were used as Control group. A physiological saline solution containing 10 % Nikkol (Nikkol: trade name, dissolving aid, supplied by Nikko Chemical Co., Ltd.) was administered intraperitoneally to the 18 mice for the consecutive 5 days after the sensitization, and ear thickness of each of these 18 mice was measured on the eighth day after the sensitization. Thereafter, 20 $\mu l$ of an olive oil solution of 1 % picryl chloride was applied to the ear of each of the mice to cause delayed-type hypersensitivity.

The remaining 56 mice were grouped into the following Example groups:

Example group ①

A group of mice to which a physiological saline solution of 10 % Nikkol containing VIE-MAN was to be administered intraperitoneally at such a dose that the amount of VIE-MAN was 10 mg/kg/day (20 mice).

Example group ②

A group of mice to which a physiological saline solution of 10 % Nikkol containing VIE-MAN was to be administered intraperitoneally at such a dose that the amount of VIE-MAN was 30 mg/kg/day (20 mice).

Example group ③

A group of mice to which a physiological saline solution of 10 % Nikkol containing VIE-MAN was to be administered intraperitoneally at such a dose that the amount of VIE-MAN was 100 mg/kg/day (18 mice).

The above pharmaceutical solutions were administered intraperitoneally at the above-described doses for the five consecutive days after the sensitization, and ear thickness of each of these mice was measured on the eighth day after the sensitization. Thereafter, 20 $\mu l$ of an olive oil solution of 1 % picryl chloride was applied to the ear of each of the mice to cause delayed-type hypersensitivity.

24 Hours after the delayed-type hypersensitivity was induced, the swelling of the ear of each of the mice of Control group and Example groups ①, ② and ③ was measured with a dial thickness gauge. On the basis of the values of the ear thickness measured before the application of the olive oil solution of 1 % picryl chloride, the swelling ratio of each of Example groups ①, ② and ③ and Control group was determined. Fig. 1 shows the results.

In Comparative Example 1, the treatment of mice in Example groups ①, ② and ③ was repeated except that methotrexate conventionally used as an immunosuppressive preparation was used in place of the VIE-MAN and that a physiological saline solution of 10 % Nikkol containing methotrexate, as a pharmaceutical solution, was administered intraperitoneally at such a dose that the amount of the methotrexate was 1 mg/kg/day, whereby delayed-type hypersensitivity was induced without causing a side effect. And, the swelling ratio of the mice was determined. Fig. 1 also shows this result as Comparative Example group ⓐ.

Fig. 1 clearly shows that the swelling ratios of Example groups ①, ② and ③ were significantly lower than that of Control group, and also shows that the delayed-type hypersensitivity was dose-dependently prevented and cured when 10, 30 and 100 mg/kg/day of VIE-MAN was administered intraperitoneally. Further, the comparison between the swelling ratio of each of Example groups ①, ② and ③ and that of Comparative Example group ⓐ shows that VIE-MAN was superior to methotrexate in the preventive and therapeutical effect on delayed-type hypersensitivity.

In addition, side effects such as a reduction in body weight, etc., were not observed in any of Example groups ①, ② and ③.

Example 2

At first, the abdominal portion of each of forty ICR male mice weighing 20 to 25 g was shaved such that the shaved portion was about 1 $cm^2$. Thereafter, 20 $\mu l$ of an ethanol solution of 7 % picryl chloride was

applied to the shaved portions for sensitization.

Fourteen out of the 40 mice were taken out for Control group, and treated in the same manner as that for Control group in Example 1, whereby delayed-type hypersensitivity was induced.

The remaining 26 mice were grouped into the following two Example groups:

Example group ④

A group of mice to which a physiological saline solution of 10 % Nikkol containing VIE-GLU, as a pharmaceutical solution, was to be administered intraperitoneally at such a dose that the amount of VIE-GLU was 100 mg/kg/day (14 mice).

Example group ⑤

A group of mice to which a physiological saline solution of 10 % Nikkol containing VIE-MAN, as a pharmaceutical solution, was to be administered intraperitoneally at such a dose that the amount of VIE-MAN was 100 mg/kg/day (12 mice).

These mice were treated in the same manner as that for Control groups ①, ② and ③ in Example 1, whereby delayed-type hypersensitivity was induced.

Thereafter, ear swelling of each of the mice of Example groups ④ and ⑤ and Control group was measured and the swelling ratio was determined in the same manner as in Example 1. Fig. 2 shows the results.

Fig. 2 clearly shows that the swelling ratios of Example groups 4 and 5 were significantly lower than that of Control group, and it has been found that VIE-GLU and VIE-MAN have an excellent preventive and therapeutical effect on delayed-type hypersensitivity.

In addition, side effects such as a reduction in body weight, etc., were not observed in any of Example groups ④ and ⑤.

Example 3 and Comparative Example 2

At first, 20 male SD rats weighing 180 to 220 g were prepared. Egg-white albumin in an amount of 1 mg per rat was prepared as an antigen to induce the IgE antibody-antigen mediating immune response. Further, a mixture of 20 mg/rat of aluminum hydroxide with 0.6 ml/rat of pertussis vaccine was prepared as an immune aid (adjuvant).

Then, the above antigen and the above adjuvant were equally administered intracutaneously into the four footpads of each rat for sensitization.

24 Hours after the sensitization, the footpad swelling of each rat was measured by volumetry to determine the swelling ratio (unit: %). Then, the rats were separated into four groups each consisting of 5 rats such that the average swelling ratio of one group was nearly equal to that of another group.

Thereafter, one of the four groups was used as Control group, and a physiological saline solution of 10 % Nikkol (trade name, dissolving aid, supplied by Nikko Chemical Co., Ltd.) was administered intraperitoneally to each rat for the 11 consecutive days after the group separation, and on 15th day after the group separation, the serum of each rat was collected, and measured for an IgE antibody amount by means of a 48-hour homologous PCA reaction (passive cutaneous anaphylaxis reaction) as an index. In addition, the IgE antibody amount is shown in terms of a maximum dilution (unit: %) of serum showing positive reaction (blue spot having a diameter of not less than 5 mm). Table 1 shows the results.

Two groups of the remaining three groups were used as Example groups ⑥ and ⑦. A physiological saline solution of 10 % Nikkol containing VIE-MAN was administered intraperitoneally to each rat of Example group ⑥ in such a dose that the amount of VIE-MAN was 30 mg/kg/day for 11 days after the group separation. And, a physiological saline solution of 10 % Nikkol containing VIE-MAN was administered intraperitoneally to each rat of Example group ⑦ every other day (number of days for administration: 6 days) in such a dose that the amount of VIE-MAN was 100 mg/kg/day for 11 days after the group separation. Then, on 15th day after the group separation, the serum of each rat was collected, and measured for an IgE antibody amount in the same manner as that for Control group. Table 1 also shows the results.

One remaining group of the four groups was used as Comparative Example group ⓑ. A physiological saline solution of 10 % Nikkol containing hydrocortisone which was an adrenocortical hormone preparation was administered intraperitoneally to each rat in such a dose that the amount of the hydrocortisone was 10 mg/kg/day for the consecutive 11 days after the group separation. Then, on 15th day after the group

separation, the serum of each rat was collected, and measured for an IgE antibody amount in the same manner as that for Example groups ⑥ and ⑦. Table 1 also shows the results.

Table 1

| | | IgE antibody amount in serum *1 | | | | | Average value |
|---|---|---|---|---|---|---|---|
| | | 1 | 2 | 3 | 4 | 5 | |
| Example 3 | VIE-MAN administered Example group ⑥ [30 mg/kg/day (total 11 days)] | 256 | 256 | 64 | 128 | 128 | 166.4 |
| | VIE-MAN administered Example group ⑦ [100 mg/kg/day (total 6 days)] | 128 | 64 | 256 | 128 | 64 | 128.0 |
| | Control group | 256 | 2,048 | 258 | 512 | 512 | 716.8 |
| Comparative Example 2 | Hydrocortisone administered Comparative Example group ⓑ [10 mg/kg/day (total 11 days)] | 512 | 1,024 | 64 | 512 | 256 | 473.6 |

*1: IgE antibody amount in serum is expressed in terms of a maximum dilution of serum showing positive reaction (blue spot having a diameter of not less than 5 mm). Numerals 1 to 5 in this column are numerals used to identify individual rats of each of the groups.

*2: Average value shows the average of IgE antibody amounts in serum as one group unit.

Table 1 clearly shows that the average value of the IgE antibody amounts of Example group ⑥ or ⑦ as a group unit was significantly lower than that of the IgE antibody amounts of Control group as a group unit, and it has been found that VIE-MAN has excellent inhibition activity against the IgE antibody production. The results of Comparative Example group ⓑ in Table 1 shows that the inhibition activity of hydrocortisone against the IgE antibody production was lower than that of VIE-MAN.

Further, side effects such as a reduction in body weight, etc., were not observed in any of Example groups ⑥ and ⑦.

In addition, the same test as above was carried out by using VIE-GLU in place of VIE-MAN and it has been confirmed that VIE-GLU also have excellent inhibition activity against the IgE antibody production.

Example 4

At first, five groups each consisting of five SD male rats weighing 180 to 220 g (total 25 rats) were prepared, and each rat was measured for a size of the subplantar region of the right hind paw. As an antigen to cause an antigen-adjuvant-induced inflammation, 0.2 mg/rat of egg-white albumin was used. Further, as an immune aid (adjuvant), a mixture consisting of 4 mg/rat of aluminum hydroxide and 0.1 ml/rat of pertussis vaccine was prepared.

One out of the five groups was used as Control group. A physiological saline solution of 10 % Nikkol (trade name, dissolving aid, supplied by Nikko Chemical Co., Ltd.) was administered intraperitoneally to each rat, and 0.5 hour after the administration of the physiological saline solution of 10 % Nikkol, the above antigen and adjuvant were administered intracutaneously into the subplantar region of the right hind paw. Then, 0.5, 1, 2, 4, 6, 12 and 24 hours after the administration of the antigen and adjuvant, the swelling on

the subplantar region of the right hind paw of each rat was measured by volumetry. And, the average of swelling ratios of the group in every measurement was determined on the basis of the measurement values of the sizes of subplantar regions of the right hind paws of the rats obtained before the administration of the physiological saline solution of 10 % Nikkol. Fig. 3 shows the results.

One of the remaining four groups was used as Example group ⑧. A physiological saline solution of 10 % Nikkol containing VIE-MAN was administered intraperitoneally into each rat of this group in such a dose that the amount of VIE-MAN was 10 mg/kg, and 0.5 hours after the administration of the physiological saline solution of 10 % Nikkol containing VIE-MAN, the above antigen and adjuvant were administered intracutaneously into the subplantar region of the right hind paw. Thereafter, the swelling on the subplantar region of the right hind paw of each rat was measured in the same manner as that for Control group, and the average of swelling ratios of the group in every measurement was determined. Fig. 3 also shows the results.

The remaining three groups were used as Comparative Example groups ⓒ, ⓓ and ⓔ, and averages of swelling ratios as a group were determined in the following manner.

Comparative Example group ⓒ

The same procedure as that for Example group ⑧ was carried out except that a physiological saline solution of 10 % Nikkol containing hydrocortisone which was an anti-inflammatory preparation (immunosuppressive preparation) in place of the physiological saline solution of 10 % Nikkol containing VIE-MAN was administered intraperitoneally in such a dose that the amount of the hydrocortisone was 10 mg/kg.

Comparative Example group ⓓ

The same procedure as that for Example group ⑧ was carried out except that a physiological saline solution of 10 % Nikkol containing diphenhydramine which was an antihistamine preparation in place of the physiological saline solution of 10 % Nikkol containing VIE-MAN was administered intraperitoneally in such a dose that the amount of the diphenhydramine was 10 mg/kg.

Comparative Example group ⓔ

The same procedure as that for Example group ⑧ was carried out except that a physiological saline solution of 10 % Nikkol containing indomethacin which was a nonsteroidal anti-inflammatory preparation (antipyretic analgesic antiphlogistic preparation) in place of the physiological saline solution of 10 % Nikkol containing VIE-MAN was administered intraperitoneally in such a dose that the amount of the indomethacin was 10 mg/kg.

Fig. 3 also shows the results of these Comparative Example groups ⓒ, ⓓ and ⓔ.

Fig. 3 clearly shows that the average of the swelling ratios of Example group 8 to which VIE-MAN had been administered was already significantly lower than that of the swelling ratios of Control group when 0.5 hour passed after the administration of the antigen and adjuvant. Thus, it has been found that VIE-MAN shows excellent inhibition activity against antigen-adjuvant-induced inflammations.

On the other hand, with regard to hydrocortisone in Comparative Example group ⓒ and diphenhydramine in Comparative Example group ⓓ, no inhibition activity was found. The indomethacin in Comparative Example group ⓔ, when used in such a high dose as 10 mg/kg, showed the significant inhibition activity at and after 2 hours of the administration of the antigen and adjuvant. However, the inhibition activity thereof was much lower than that of VIE-MAN.

These results show that VIE-MAN also has an excellent anti-inflammatory activity against inflammations which are difficult to prevent and cure with a nonsteroidal anti-inflammatory preparation and a steroidal anti-inflammatory preparation.

Example 5

At first, four groups each consisting of five male SD rats weighing 180 to 220 g (total 20 rats) were prepared, and each rat was measured for a size of the subplantar region of the right hind paw. As an antigen to cause an antigen- adjuvant-induced inflammation, 0.2 mg/rat of egg-white albumin was prepared. Further, as an immune aid (adjuvant), a mixture consisting of 4 mg/rat of aluminum hydroxide and 0.1 ml/rat of pertussis vaccine was prepared.

One out of the four groups was used an Control group, and the procedure for Control group in Example 4 was repeated to determine the average of the swelling ratios as a group unit. Fig. 4 shows the result.

The remaining three groups were used as Example groups ⑨, ⑩ and ⑪, and averages of swelling ratios as a group unit were determined in the following manner.

Example group ⑨

The procedure for Control group was repeated except that a physiological saline solution of 10 % Nikkol containing VIE-GLU in place of the physiological saline solution of 10 % Nikkol was administered intraperitoneally in such a dose that the amount of VIE-GLU was 10 mg/kg.

Example group ⑩

The procedure for Control group was repeated except that a physiological saline solution of 10 % Nikkol containing VIE-GAL in place of the physiological saline solution of 10 % Nikkol was administered intraperitoneally in such a dose that the amount of VIE-GAL was 10 mg/kg.

Example group ⑪

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 10 mg/kg.

Fig. 4 also shows the results of these Example groups ⑨, ⑩ and ⑪.

Fig. 4 clearly shows that the average of the swelling ratios of Example group ⑨ to which VIE-GLU had been administered was already significantly lower than that of the swelling ratios of Control group when 0.5 hour passed after the administration of the antigen and adjuvant, and it has been found that VIE-GLU is almost as superior as VIE-MAN in the inhibition activity against antigen-adjuvant-induced inflammations. Further, the average of the swelling ratios of Example group ⑩ to which VIE-GAL had been administered was already significantly lower than that of the swelling ratios of Control group at 0.5 hour after the administration of the antigen and adjuvant, and it has been found that VIE-GAL is also superior in the inhibition activity against antigen-adjuvant-induced inflammations.

Example 6

24 male SD rats weighing 180 to 220 g were used, and measured for a size of the subplantar region of the right hind paw. As a substance to cause adjuvant arthritis, 0.05 ml/rat of Freund's complete adjuvant [ADJUVANT COMPLETEFREUND, trade name, supplied by Difco] was used.

Then, the above Freund's complete adjuvant was administered intracutaneously into the subplantar region of right hind paw of each rat, and 24 hours after the administration, the swelling on the right hind paw of each rat was measured by volumetry to determine a swelling ratio. The above rats were separated into four groups each consisting of 6 rats such that the average swelling ratio of one group was nearly equal to that of another group.

Thereafter, one out of the four groups was used as Control group. A physiological saline solution of 10 % Nikkol was administered intraperitoneally to each rat of this group for the consecutive 22 days after the group separation, and the swelling on the right hind paw was measured by volumetry every day after the administration of the physiological saline solution of 10 % Nikkol. The average of swelling ratios as a group for each day was determined on the basis of the values of the sizes of the subplantar regions of the right hind paws obtained by the measurement prior to the administration of the Freund's complete adjuvant. Fig. 5 shows the results.

Two of the remaining three groups were used as Example groups ⑫ and ⑬, and the average of swelling ratios of each group was determined in the following manner.

Example group ⑫

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 10 mg/kg/day.

Example group ⑬

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 30 mg/kg/day.

Fig. 5 also shows the results of the Example groups ⑫ and ⑬.

The remaining one group was used as Comparative Example group ⓕ. The procedure for Example groups ⑫ and ⑬ was repeated except that a physiological saline solution of 10 % Nikkol containing indomethacin which was a nonsteroidal anti-inflammatory preparation (antipyretic analgesic antiphlogistic preparation) in place of the physiological saline solution of 10 % Nikkol was administered intraperitoneally in such a dose that the amount of the indomethacin was 10 mg/kg/day, and the average of swelling ratios as a group was determined. Fig. 5 also shows the results of Comparative Example group ⓕ.

Fig. 5 clearly shows that the average of the swelling ratios of each of Example groups ⑫ and ⑬ to which VIE-MAN had been administered was significantly lower than that of the swelling ratios of Control group, and it has been found that VIE-MAN exhibited excellent inhibition activity against both of the acute inflammation, which occurred before the 14th day after the administration of the Freund's complete adjuvant, and adjuvant arthritis, which occurred after 17th day, depending upon the dose.

On the other hand, it has been found that the indomethacin in Comparative Example group ⓕ, when used in such as high dose as 10 mg/kg/day, showed inhibitory activity against both acute inflammation and adjuvant arthritis. However, the activity thereof was much lower than that of VIE-MAN.

Example 7

Six groups each consisting of five male SD rats weighing 180 to 220 g were used (total 30 rats), and each rat was measured for a size of the subplantar region1 of the right hind paw.

In order to induce carrageenin-induced inflammation, 0.1 ml/rat of a physiological saline solution of 1 % λ-carrageenin was prepared.

One out of the six groups was used as Control group. A physiological saline solution of 10 % Nikkol was administered intraperitoneally to each rat of this group, and 0.5 hours after the administration of the physiological saline solution of 10 % Nikkol, 0.1 ml of the physiological saline solution of 1 % λ-carrageenin was administered intracutaneously into the subplantar region of the right hind paw of each rat. Then, 0.5, 1, 2, 3 and 4 hours after the administration of 1 ml of the physiological saline solution of 1 % λ-carrageenin, the swelling on the subplantar region of the right hind paw of each rat was measured by volumetry, arid the average of swelling ratios of the group in every measurement was determined on the basis of the measurement values of the sizes of subplantar regions of the right hind paws of the rats obtained before the administration of the physiological saline solution of 10 % Nikkol solution. Fig. 6 shows the results.

Three out of the remaining five groups were used as Example groups ⑭, ⑮ and ⑯, and the average of swelling ratio of each group was determined in the following manner.

Example group ⑭

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 10 mg/kg.

Example group ⑮

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 30 mg/kg.

Example group ⑯

The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 100 mg/kg.

Fig. 6 also shows the results of these Example groups ⑭, ⑮ and ⑯.

The remaining two groups were used as Comparative Example groups ⓖ and ⓗ, and the average of

swelling ratios of each group was determined in the following manner.

Comparative Example group ⓖ

The procedure for Example groups ⑭, ⑮ and ⑯ was repeated except that a physiological saline solution of 10 % Nikkol containing cortisone acetate which was a steroidal anti-inflammatory preparation (immunosuppressive preparation) in place of the physiological saline solution of 10 % Nikkol containing VIE-MAN was administered intraperitoneally in such a dose that the amount of the cortisone acetate was 10 mg/kg.

Comparative Example group ⓗ

The procedure for Example groups ⑭, ⑮ and ⑯ was repeated except that a physiological saline solution of 10 % Nikkol containing indomethacin which was a nonsteroidal anti-inflammatory preparation (antipyretic analgesic antiphlogistic preparation) in place of the physiological saline solution of 10 % Nikkol containing VIE-MAN was administered intraperitoneally in such a dose that the amount of the indomethacin was 10 mg/kg.

Fig. 6 also shows the results of these Comparative Example groups ⓖ and ⓗ.

Fig. 6 clearly shows that the average of swelling ratios of each of Example groups ⑭, ⑮ and ⑯ to which VIE MAN had been administered was already significantly lower than that of swelling ratios of Control group when 0.5 hour passed after the administration of the physiological saline solution of 1 % λ-carrageenin, and it has been thus found that VIE-MAN exhibits excellent inhibition activity against carrageenin-induced inflammation depending upon its dose.

On the other hand, it was found that cortisone acetate in Comparative Example group ⓖ and indomethacin in Comparative Example group ⓗ, when used in such a high dose as 10 mg/kg, showed significant activity at and after 0.5 hour of the administration of the physiological saline solution of 1 % λ-carrageenin. However, the inhibition activity thereof was lower than that of VIE-MAN.

Example 8

15 male SD rats weighing 180 to 220 g were used. These rats were fasted for 24 hours, and separated to three groups each consisting of 5 rats.

Then, one group was used as Control group. A physiological saline solution of 10 % Nikkol was administered intraperitoneally, and the rats were sacrificed with ether 16 hours after the administration. The stomach of each rat was removed, and after 10 ml of physiological saline was injected into the stomachs, the stomachs were fixed by immersing them in a 1 % formalin for 10 minutes. Each stomach was dissected along the greater curvatura, and the length (mm) of mucosal ulcer which occurred in the glandular portion was measured under an anatomical microscope (x 10). The average of the ulcer lengths of the five rats was taken as an ulcer index.

One out of the remaining two groups was used as Example group ⑰. The procedure for Control group was repeated except that a physiological saline solution of 10 % Nikkol containing VIE-MAN in place of the physiological saline solution of 10 % Nikkol was administered intraperitoneally in such a dose that the amount of VIE-MAN was 300 mg/kg, and the ulcer index was determined.

The remaining one group was used as Comparative Example group ⓘ. The procedure for Example group 17 was repeated except that a physiological saline solution of 10 % Nikkol containing indomethacin, which was a nonsteroidal anti-inflammatory preparation (antipyretic analgesic antiphlogistic preparation), in place of the physiological saline solution of 10 % Nikkol was administered intraperitoneally in such a dose that the amount of the indomethacin was 20 mg/kg, and the ulcer index was determined.

Fig. 7 shows the results.

As is clearly shown in Fig. 7, similarly to Control group, no mucosal ulcer was observed in Example group ⑰ to which VIE-MAN had been administered, and it has been found that VIE-MAN has no side effect on the digestive system.

On the other hand, in Comparative Example group ⓘ to which indomethacin had been administered, the ulcer index was as high as about 30 mm although the dose thereof was as small as 20 mg/kg as compared with that of VIE-MAN, and indomethacin accordingly has a high side effect on the digestive system.

In addition, the test on the side effect using VIE-GLU and VIE-GAL in place of VIE-MAN also showed nearly similar results to that of the test on VIE-MAN.

The above description can be summarized as follows.

(1) The preventive and therapeutical preparation for delayed-type hypersensitivity, provided by the present invention, has an excellent preventive and therapeutical effect over, and exhibits a lower side effect than, conventional pharmaceutical preparations used as a preventive and therapeutical preparation for delayed-type hypersensitivity.

Therefore, the working of the present invention makes it possible to prevent and cure delayed-type hypersensitivity more effectively.

(2) The IgE antibody production inhibiting preparation of the present invention has the excellent inhibition activity against IgE antibody production.

Therefore, the working of the present invention makes it possible to radically cure diseases caused by an IgE antibody-antigen mediating immune response.

(3) The anti-inflammatory preparation of the present invention exhibits excellent anti-inflammatory activity against a wide range of inflammations including inflammations difficult to prevent and cure with conventional nonsteroidal anti-inflammatory preparations and conventional steroidal anti-inflammatory preparations, and has no side effect on the digestive system.

Therefore, the working of the present invention makes it possible to prevent and cure various inflammations safely and effectively.

## Claims

1. A preventive and therapeutical preparation for delayed-type hypersensitivity, which contains, as an active component, at least one substance selected from α-tocopheryl glycosides.

2. A preventive and therapeutical preparation for delayed-type hypersensitivity according to claim 1, wherein the α-tocopheryl glycoside is dl-α-tocopheryl-α-D-mannoside or dl-α-tocopheryl-β-D-glucoside.

3. An immunoglobulin E antibody production inhibiting preparation, which contains, as an active component, at least one substance selected from α-tocopheryl glycosides.

4. An immunoglobulin E antibody production inhibiting preparation according to claim 3, wherein the α-tocopheryl glycoside is dl-α-tocopheryl-α-D-mannoside or dl-α-tocopheryl-β-D-glucoside.

5. An anti-inflammatory preparation, which contains, as an active component, at least one substance selected from α-tocopheryl glycosides.

6. An anti-inflammatory preparation according to claim 5, wherein the α-tocopheryl glycoside is selected from the group consisting of dl-α-tocopheryl-α-D-mannoside, dl-α-tocopheryl-β-D-glucoside and dl-α-tocopheryl-β-D-galactoside.

FIG. 1

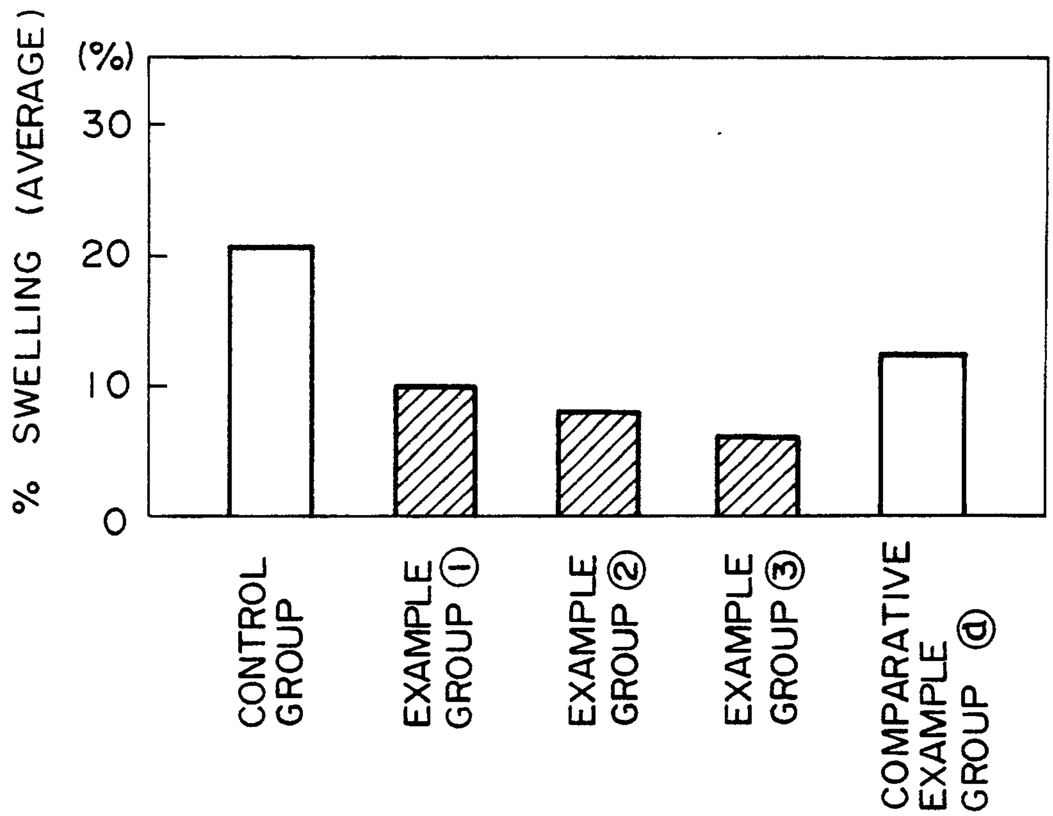
% SWELLING (AVERAGE)
(%)
30
20
10
0
CONTROL GROUP
EXAMPLE GROUP ①
EXAMPLE GROUP ②
EXAMPLE GROUP ③
COMPARATIVE EXAMPLE GROUP ⓓ

FIG. 2

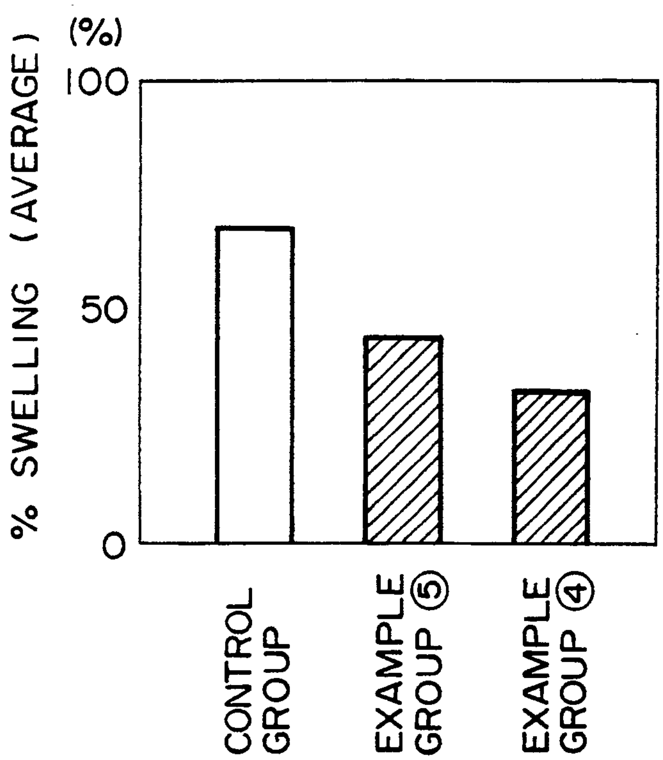
% SWELLING (AVERAGE)
(%)
100
50
0
CONTROL GROUP
EXAMPLE GROUP ⑤
EXAMPLE GROUP ④

FIG. 3

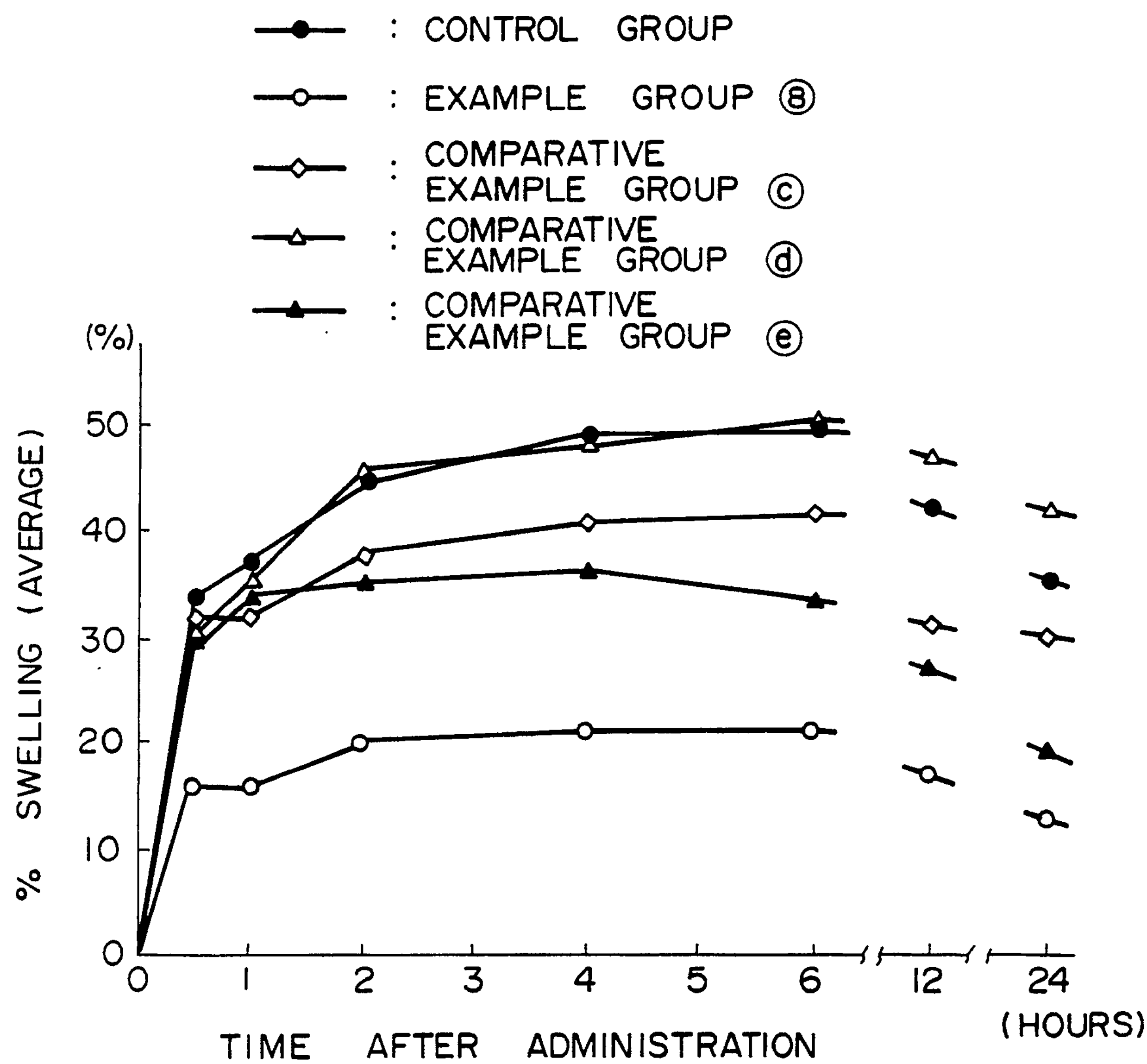

: CONTROL GROUP
: EXAMPLE GROUP ⑧
: COMPARATIVE EXAMPLE GROUP ⓒ
: COMPARATIVE EXAMPLE GROUP ⓓ
: COMPARATIVE EXAMPLE GROUP ⓔ
(%)
% SWELLING (AVERAGE)
0
10
20
30
40
50
0
1
2
3
4
5
6
12
24
(HOURS)
TIME AFTER ADMINISTRATION

FIG.4

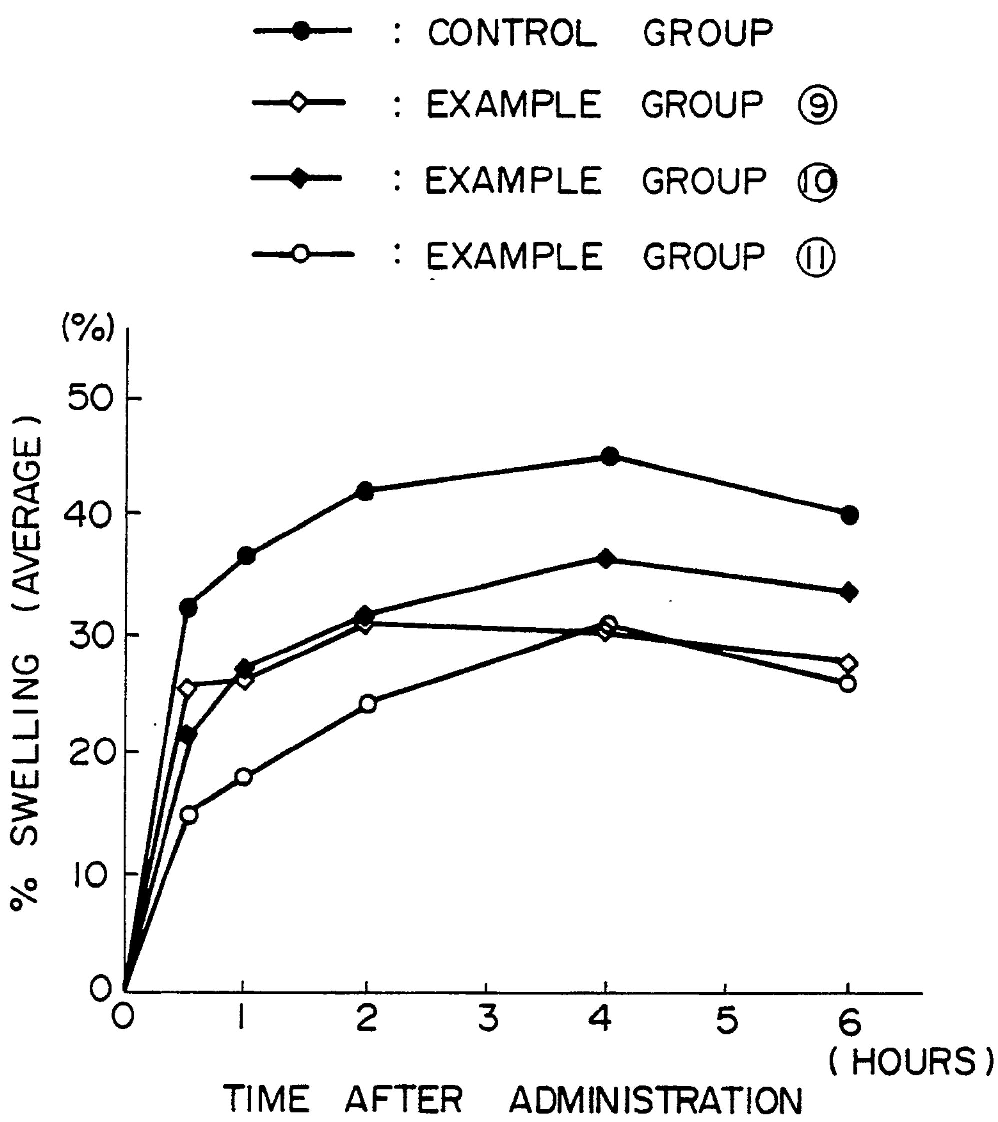
: CONTROL GROUP
: EXAMPLE GROUP ⑨
: EXAMPLE GROUP ⑩
: EXAMPLE GROUP ⑪
(%)
50
40
30
20
10
0
% SWELLING (AVERAGE)
0
1
2
3
4
5
6
(HOURS)
TIME AFTER ADMINISTRATION

FIG. 5

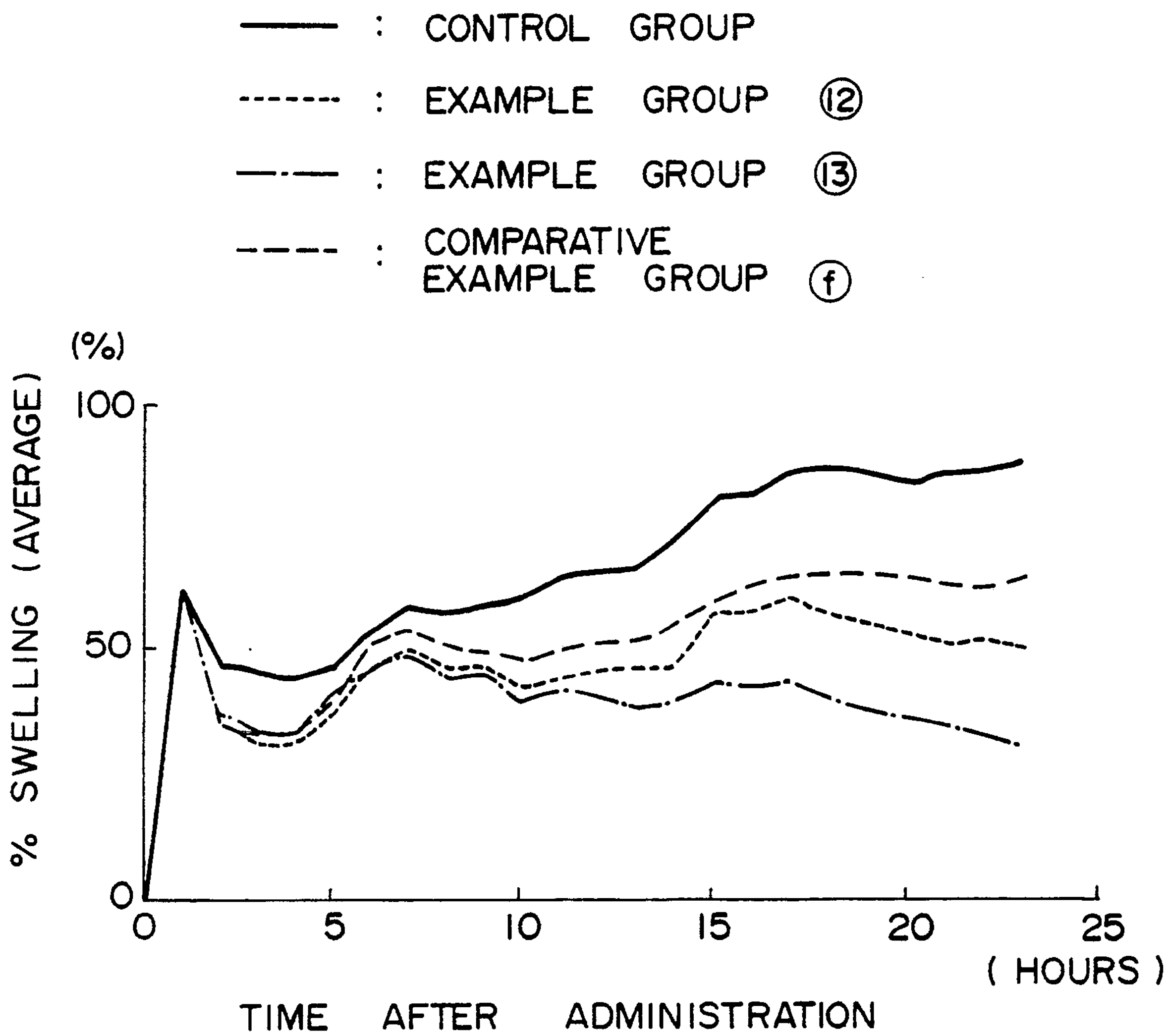
: CONTROL GROUP
: EXAMPLE GROUP ⑫
: EXAMPLE GROUP ⑬
: COMPARATIVE EXAMPLE GROUP ⓕ
(%)
% SWELLING (AVERAGE)
100
50
0
0
5
10
15
20
25
( HOURS )
TIME AFTER ADMINISTRATION

FIG.6

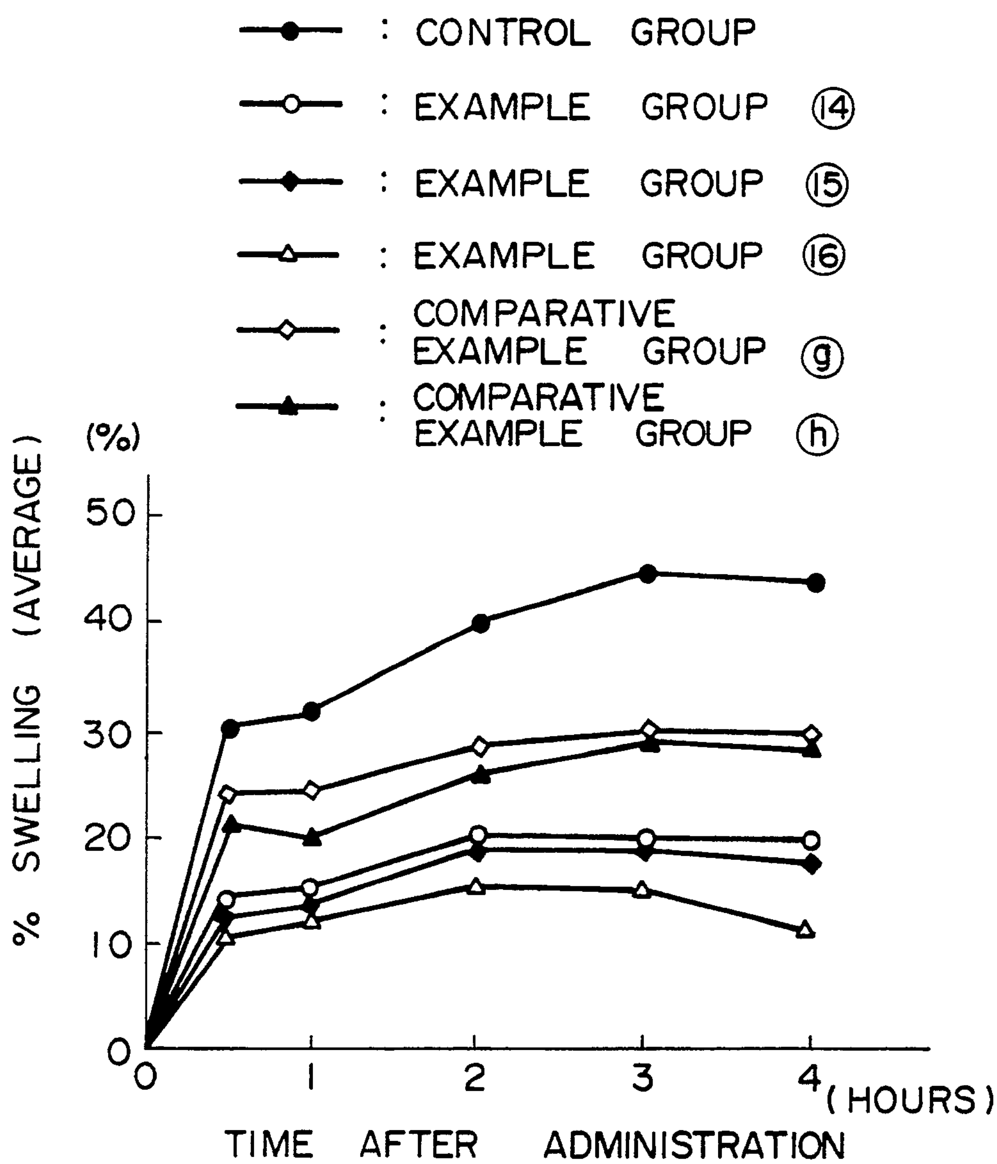
CONTROL GROUP
EXAMPLE GROUP ⑭
EXAMPLE GROUP ⑮
EXAMPLE GROUP ⑯
COMPARATIVE EXAMPLE GROUP ⓖ
COMPARATIVE EXAMPLE GROUP ⓗ
(%)
% SWELLING (AVERAGE)
50
40
30
20
10
0
0
1
2
3
4
(HOURS)
TIME AFTER ADMINISTRATION

FIG. 7

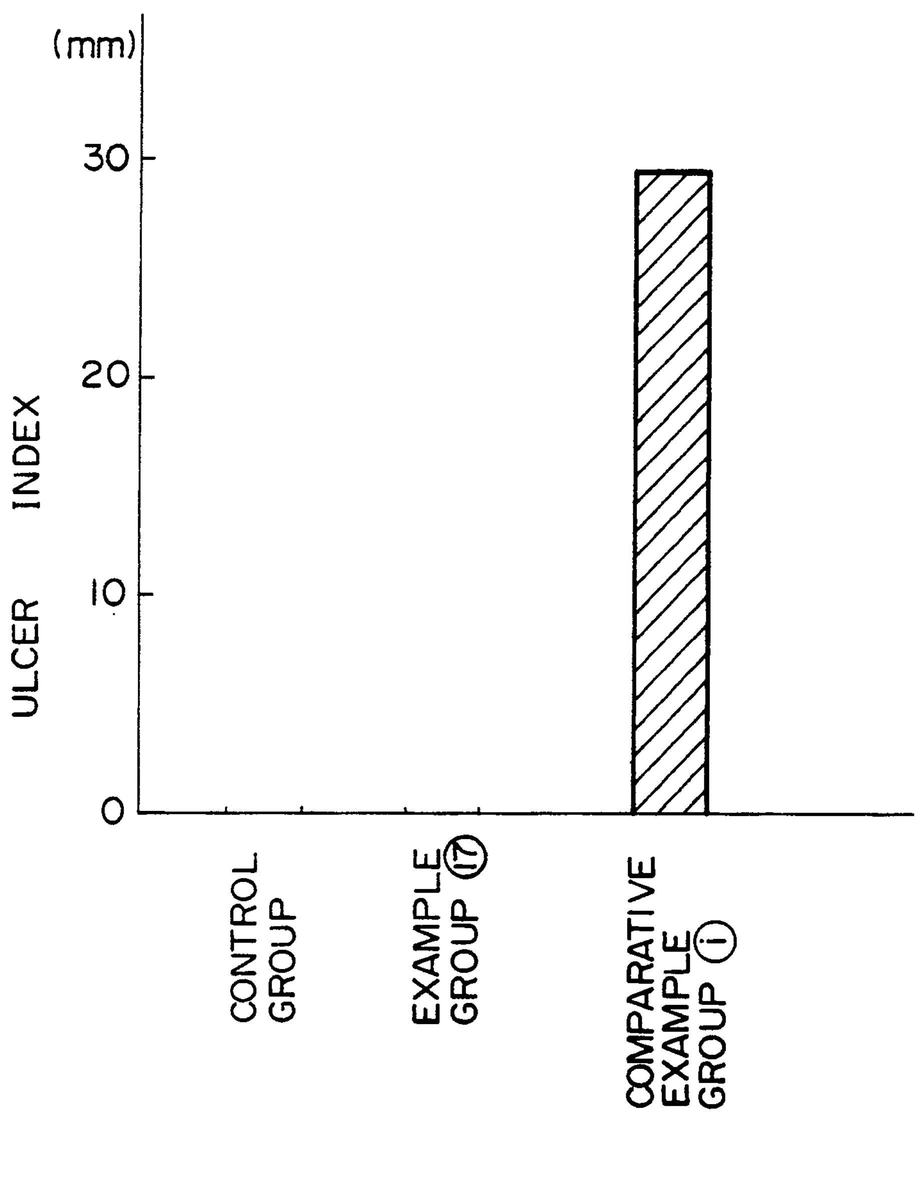
(mm)
30
20
10
0
ULCER INDEX
CONTROL GROUP
EXAMPLE GROUP ⑰
COMPARATIVE EXAMPLE GROUP ⓘ

# INTERNATIONAL SEARCH REPORT

International Application No PCT/JP90/01450

**I. CLASSIFICATION OF SUBJECT MATTER** (if several classification symbols apply, indicate all) [6]

According to International Patent Classification (IPC) or to both National Classification and IPC

Int. $Cl^5$ A61K31/70//C07H17/065

**II. FIELDS SEARCHED**

Minimum Documentation Searched [7]

| Classification System | Classification Symbols |
|---|---|
| IPC | C07H17/06-065, A61K31/355, 31/70 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 61-130229 (Sunstar Inc.), June 18, 1986 (18. 06. 86) & Chemical Abstracts 105(20):178449c | 1-6 |
| X | EP, A2, 169716 (Sunstar Inc.), January 29, 1986 (29. 01. 86) & JP, A, 61-30594 & Chemical Abstracts 104(24):213266u | 1-6 |
| X | JP, A, 60-56994 (Sunstar Inc.), April 2, 1985 (02. 04. 85) & Chemical Abstracts 104(5):34290a | 1-6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 25, 1991 (25. 01. 91) | February 12, 1991 (12. 02. 91) |

| International Searching Authority | Signature of Authorized Officer |
|---|---|
| Japanese Patent Office | |